# EUROPEAN PATENT SPECIFICATION

(11) **EP 4 588 916 B1**
(45) Date of publication and mention of the grant of the patent: **04.11.2026**
(21) Application number: 23864726.7
(22) Date of filing: 13.09.2023
(51) Int. Cl.: C07D 405/14, A61K 31/501, A61P 35/00

(54) **CRYSTAL FORM OF NAPHTHOFURAN-SUBSTITUTED GLUTARIMIDE COMPOUND, AND PREPARATION METHOD THEREFOR AND USE THEREOF**
KRISTALLINE FORM EINER NAPHTHOFURANSUBSTITUIERTEN GLUTARIMIDVERBINDUNG SOWIE HERSTELLUNGSVERFAHREN DAFÜR UND VERWENDUNG DAVON
FORME CRISTALLINE D'UN COMPOSÉ GLUTARIMIDE SUBSTITUÉ PAR NAPHTOFURANE, SON PROCÉDÉ DE PRÉPARATION ET SON UTILISATION

(30) Priority: 14.09.2022 CN 202211119711; 29.08.2023 CN 202311105461
(43) Date of publication of application: 23.07.2025
(73) Proprietor: Shanghai Qilu Pharmaceutical Research and Development Centre Ltd., Shanghai 201203 (CN)
(72) Inventor: LI, Junmiao, Shanghai 200131 (CN); SUN, Lanbao, Shanghai 200131 (CN); LEI, Maoyi, Shanghai 200131 (CN); LUO, Yunfu, Shanghai 200131 (CN)
(74) Representative: De Clercq & Partners
(86) International application number: PCT/CN2023/118588
(87) International publication number: WO 2024/055994

(56) References cited:
- EP-A1- 3 660 004
- WO-A1-2020/048546
- WO-A1-2020/048547
- WO-A1-2021/185291
- WO-A1-2022/194221
- CN-A- 110 506 039

## Description

The present invention claims the right of the following priorities:
CN2022111197111, application date: September 14, 2022;
CN2023111054610, application date: August 29, 2023.

### TECHNICAL FIELD

The present disclosure relates to a crystal form of a naphthofuran-substituted glutarimide compound, a preparation method therefor, and a use thereof. It specifically relates to a crystal form of compound 1 and a preparation therefor.

### BACKGROUND

As androgen receptor signaling is known to play a crucial role in the pathogenesis of prostate cancer and is involved in the development of other androgen receptor-positive cancers, inhibition of androgen receptor signaling with an anti-androgen that antagonizes the androgen receptor has been used, or is proposed to be used, in the treatment of prostate cancer.

AR gene encodes the androgen receptor protein, whose ligands are mainly testosterone and dihydrotestosterone. The receptor is widely distributed in most organs and tissues of the body, mediating the biological effects of androgen. Androgen receptor can bind to heat shock protein (Hsp) in the cytoplasm. When androgen binds to an androgen receptor, the receptor is activated, and the heat shock protein dissociates. The androgen receptor forms a dimer which enters the nucleus, binds to androgen response element (ARE) on the DNA, and initiates the transcription and expression of a series of genes downstream of the element, including genes such as prostate-specific antigen (PSA), prostatic acid phosphatase (PAP), and cyclin-dependent kinase (CDK) inhibitor p21WAF1/CIPI, which eventually leads to cell differentiation and promotes the development of tissues and organs. Androgen has the function of maintaining prostate growth and development. In an androgen-free environment, prostate cells will undergo spontaneous apoptosis, whereas in an environment with normal androgen level, prostate cells can continue to grow and differentiate. Therefore, it has been suggested that the excessive secretion of androgen and the over-responsiveness of androgen receptor contribute to the uninhibited growth of prostate cells, which are risk factors for the development of prostate cancer.

Prostate cancer (PCa) is one of the most commonly diagnosed non-skin cancers among men in the United States. It is the second most common cause of cancer death, with more than 200,000 new cases and more than 30,000 deaths in the United States each year. Androgen deprivation therapy (ADT) is the standard treatment for advanced PCa. Patients with advanced PCa undergo ADT via luteinizing hormone-releasing hormone (LHRH) agonists, LHRH antagonists, or bilateral orchiectomy. Despite an initial response to ADT, disease progression is inevitable and the cancer emerges as castration-resistant prostate cancer (CRPC). Up to 30% of patients with prostate cancer who receive primary treatment with radiation or surgery will develop metastatic diseases within 10 years of primary treatment. Each year, about 50,000 patients will develop metastatic disease, called metastatic CRPC (mCRPC).

Proteolysis targeting chimera (PROTAC) is a technique that applies the ubiquitin-proteasome system to target specific proteins and induce their intracellular degradation. The ubiquitin-proteasome system is the main pathway for intracellular protein degradation, mainly responsible for the removal of denatured, mutated, or harmful proteins from the cell as its normal physiological functions. 80% or more of intracellular protein degradation is dependent on the ubiquitin-proteasome system. PROTAC utilizes the cell's own protein destruction mechanism to remove specific targeted proteins from the cell.

The present disclosure describes compounds, including compositions comprising the same, whose function is to recruit endogenous proteins to E3 ubiquitin ligases such as cereblon (CRBN) E3 ubiquitin ligases for ubiquitination and subsequent degradation, as well as methods of use of the same. In particular, the present disclosure provides a bi-functional or proteolysis targeting chimera (PROTAC) compound, which has been found to act as a modulator of targeted ubiquitination and androgen receptor (AR) degradation.

EP3660004 discloses PROTACs targeting the androgen receptor for the treatment of prostate cancer.

### CONTENT OF THE PRESENT INVENTION

The present disclosure provides a crystal form A of compound 1 as shown herein, 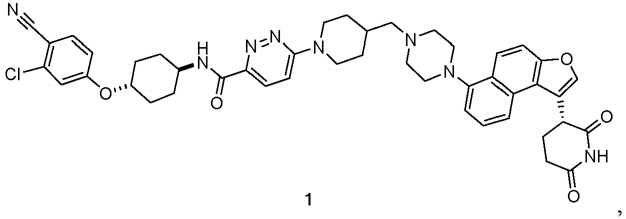 wherein the crystal form A has an X-ray powder diffraction pattern comprising characteristic diffraction peaks at the following 2θ angles: 17.521±0.200°, 19.495±0.200°, 20.531±0.200°, 20.956±0.200°, and 22.271±0.200°.

In some embodiments of the present disclosure, the X-ray powder diffraction pattern for the crystal form A of compound 1, represented by 2θ angles, comprises at least 5, 6, 7, or 8 diffraction peaks selected from the following: 9.530±0.200°, 12.135±0.200°, 12.784±0.200°, 17.521±0.200°, 19.495±0.200°, 20.531±0.200°, 20.956±0.200°, and 22.271±0.200°.

In some embodiments of the present disclosure, the X-ray powder diffraction pattern for the crystal form A of compound 1 comprises characteristic diffraction peaks at the following 2θ angles: 9.530±0.200°, 12.135±0.200°, 12.784±0.200°, 17.521±0.200°, 19.495±0.200°, 20.531±0.200°, 20.956±0.200°, and 22.271±0.200°.

In some embodiments of the present disclosure, the X-ray powder diffraction pattern for the crystal form A of compound 1, represented by 2θ angles, comprises at least 10, 11, 12, or 13 diffraction peaks selected from the following: 9.530±0.200°, 12.135±0.200°, 12.784±0.200°, 16.622±0.200°, 16.973±0.200°, 17.521±0.200°, 17.858±0.200°, 18.617±0.200°, 19.495±0.200°, 20.531±0.200°, 20.956±0.200°, 22.271±0.200°, and 22.815±0.200°.

In some embodiments of the present disclosure, the X-ray powder diffraction pattern for the crystal form A of compound 1 comprises characteristic diffraction peaks at the following 2θ angles: 9.530±0.200°, 12.135±0.200°, 12.784±0.200°, 16.622±0.200°, 17.521±0.200°, 18.617±0.200°, 19.495±0.200°, 20.531±0.200°, 20.956±0.200°, 22.271±0.200°, and 22.815±0.200°.

In some embodiments of the present disclosure, the X-ray powder diffraction pattern for the crystal form A of compound 1 comprises characteristic diffraction peaks at the following 2θ angles: 9.530±0.200°, 12.135±0.200°, 12.784±0.200°, 16.622±0.200°, 17.521±0.200°, 17.858±0.200°, 18.617±0.200°, 19.495±0.200°, 20.531±0.200°, 20.956±0.200°, 22.271±0.200°, and 22.815±0.200°.

In some embodiments of the present disclosure, the X-ray powder diffraction pattern for the crystal form A of compound 1 comprises characteristic diffraction peaks at the following 2θ angles: 9.530±0.200°, 12.135±0.200°, 12.784±0.200°, 16.622±0.200°, 16.973±0.200°, 17.521±0.200°, 17.858±0.200°, 18.617±0.200°, 19.495±0.200°, 20.531±0.200°, 20.956±0.200°, 22.271±0.200°, and 22.815±0.200°.

In some embodiments of the present disclosure, the X-ray powder diffraction pattern for the crystal form A of compound 1 comprises characteristic diffraction peaks at the following 2θ angles: 4.358°, 9.530°, 12.135°, 12.784°, 14.166°, 15.597°, 16.622°, 16.973°, 17.521°, 17.858°, 18.617°, 19.495°, 20.531°, 20.956°, 22.271°, 22.815°, 24.711°, 25.955°, 26.976°, 27.682°, 28.728°, 30.119°, 31.329°, 33.598°, and 35.500°.

In some embodiments of the present disclosure, the crystal form A of compound 1 has an X-ray powder diffraction pattern (XRPD) with peak positions and relative intensities of diffraction peaks as shown in Table 1:

**Table 1. Peak positions and relative intensities of diffraction peaks in XRPD pattern for crystal form A**

| **No.** | **2θ angle (°)** | **Relative intensity (%)** | **No.** | **2θ angle (°)** | **Relative intensity (%)** |
|---|---|---|---|---|---|
| 1 | 4.358 | 8.60 | 14 | 20.956 | 92.92 |
| 2 | 9.530 | 35.06 | 15 | 22.271 | 87.21 |
| 3 | 12.135 | 37.64 | 16 | 22.815 | 11.75 |
| 4 | 12.784 | 43.97 | 17 | 24.711 | 7.85 |
| 5 | 14.166 | 13.90 | 18 | 25.955 | 7.59 |
| 6 | 15.597 | 8.74 | 19 | 26.976 | 9.35 |
| 7 | 16.622 | 25.43 | 20 | 27.682 | 8.49 |
| 8 | 16.973 | 15.78 | 21 | 28.728 | 4.97 |
| 9 | 17.521 | 100.00 | 22 | 30.119 | 4.64 |
| 10 | 17.858 | 16.02 | 23 | 31.329 | 3.18 |
| 11 | 18.617 | 18.45 | 24 | 33.598 | 3.32 |
| 12 | 19.495 | 49.67 | 25 | 35.500 | 3.60 |
| 13 | 20.531 | 80.26 | | | |

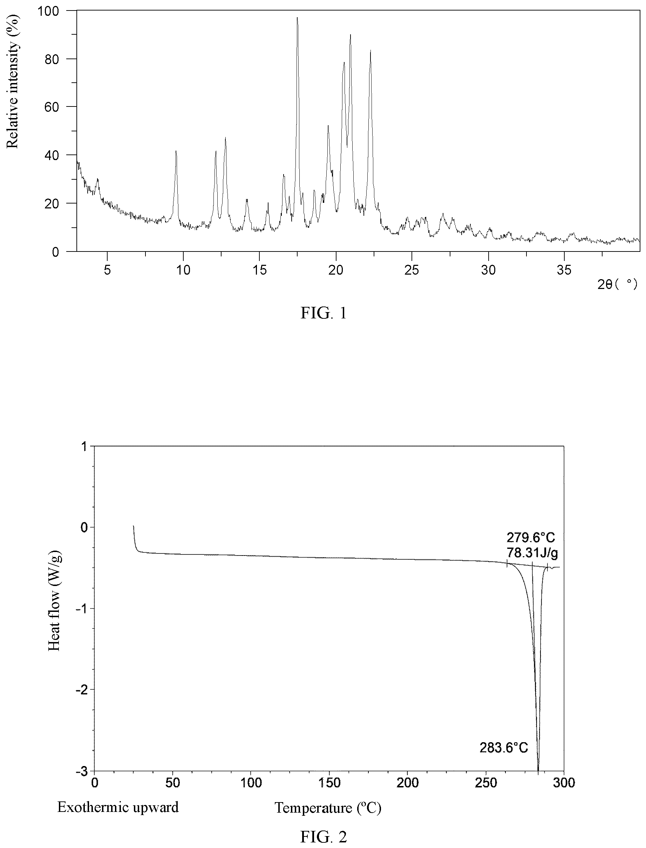

In some embodiments of the present disclosure, the crystal form A of compound 1 has an XRPD pattern as shown in FIG. 1.

In some embodiments of the present disclosure, the crystal form A of compound 1 has a differential scanning calorimetry (DSC) curve comprising an endothermic peak with an onset at 279.6°C±3.0°C.

In some embodiments of the present disclosure, the crystal form A of compound 1 has a DSC pattern as shown in FIG. 2.

In some embodiments of the present disclosure, the crystal form A of compound 1 has a thermogravimetric analysis (TGA) curve with a weight loss of 0.59% at 150.0°C±3.0°C.

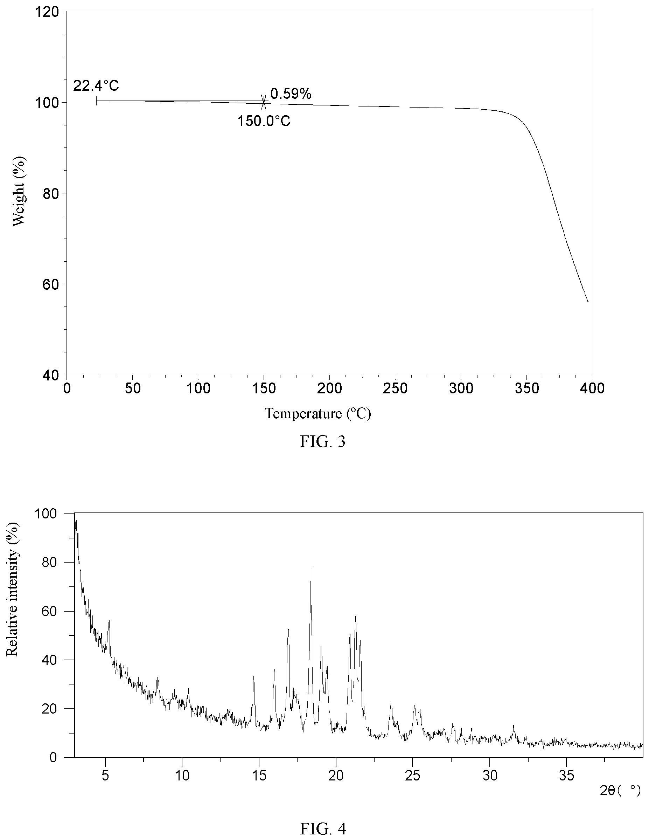

In some embodiments of the present disclosure, the crystal form A of compound 1 has a TGA pattern as shown in FIG. 3.

The present disclosure provides a crystal form B of compound 1 as shown herein, 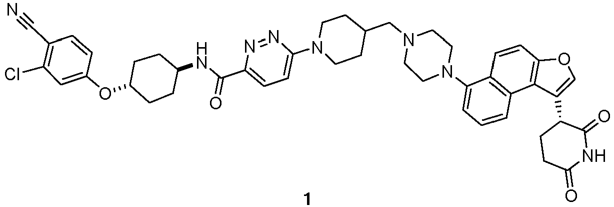 wherein the crystal form B has an X-ray powder diffraction pattern comprising characteristic diffraction peaks at the following 2θ angles: 16.923±0.200°, 18.394±0.200°, and 21.271±0.200°.

In some embodiments of the present disclosure, the X-ray powder diffraction pattern for the crystal form B of compound 1, represented by 2θ angles, comprises at least 5, 6, 7, or 8 diffraction peaks selected from the following: 16.039±0.200°, 16.923±0.200°, 18.394±0.200°, 19.070±0.200°, 19.445±0.200°, 20.915±0.200°, 21.271±0.200°, and 21.599±0.200°.

In some embodiments of the present disclosure, the X-ray powder diffraction pattern for the crystal form B of compound 1 comprises characteristic diffraction peaks at the following 2θ angles: 16.039±0.200°, 16.923±0.200°, 18.394±0.200°, 19.070±0.200°, 19.445±0.200°, 20.915±0.200°, 21.271±0.200°, and 21.599±0.200°.

In some embodiments of the present disclosure, the X-ray powder diffraction pattern for the crystal form B of compound 1, represented by 2θ angles, comprises at least 10, 11, 12, or 13 diffraction peaks selected from the following: 5.222±0.200°, 10.412±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, 23.951±0.200°, and 25.463±0.200°.

In some embodiments of the present disclosure, the X-ray powder diffraction pattern for the crystal form B of compound 1 comprises characteristic diffraction peaks at the following 2θ angles: 5.222±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, and 23.951±0.200°.

In some embodiments of the present disclosure, the X-ray powder diffraction pattern for the crystal form B of compound 1 comprises characteristic diffraction peaks at the following 2θ angles: 5.222±0.200°, 10.412±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, and 23.951±0.200°.

In some embodiments of the present disclosure, the X-ray powder diffraction pattern for the crystal form B of compound 1 comprises characteristic diffraction peaks at the following 2θ angles: 5.222±0.200°, 10.412±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, 23.951±0.200°, and 25.463±0.200°.

In some embodiments of the present disclosure, the X-ray powder diffraction pattern for the crystal form B comprises characteristic diffraction peaks at the following 2θ angles: 5.222°, 8.368°, 10.412°, 13.031°, 14.668°, 16.039°, 16.923°, 18.394°, 19.070°, 19.445°, 20.915°, 21.271°, 21.599°, 23.591°, 25.463°, 27.635°, and 31.559°.

In some embodiments of the present disclosure, the crystal form B of compound 1 has an X-ray powder diffraction pattern (XRPD) with peak positions and relative intensities of diffraction peaks as shown in Table 2:

**Table 2. Peak positions and relative intensities of diffraction peaks in XRPD pattern for crystal form B**

| **No.** | **2θ angle (°)** | **Relative intensity (%)** | **No.** | **2θ angle (°)** | **Relative intensity (%)** |
|---|---|---|---|---|---|
| 1 | 5.222 | 20.01 | 10 | 19.445 | 35.67 |
| 2 | 8.368 | 8.60 | 11 | 20.915 | 61.91 |
| 3 | 10.412 | 12.69 | 12 | 21.271 | 71.56 |
| 4 | 13.031 | 5.89 | 13 | 21.599 | 56.86 |
| 5 | 14.668 | 30.32 | 14 | 23.591 | 21.28 |
| 6 | 16.039 | 36.83 | 15 | 25.463 | 17.67 |
| 7 | 16.923 | 62.34 | 16 | 27.635 | 9.10 |
| 8 | 18.394 | 100.00 | 17 | 31.559 | 7.97 |
| 9 | 19.070 | 49.72 | | | |

In some embodiments of the present disclosure, the crystal form B of the solvate has an XRPD pattern as shown in FIG. 4.

In some embodiments of the present disclosure, the crystal form B of the solvate has a differential scanning calorimetry (DSC) curve comprising an endothermic peak with an onset at 290.2°C±3.0°C.

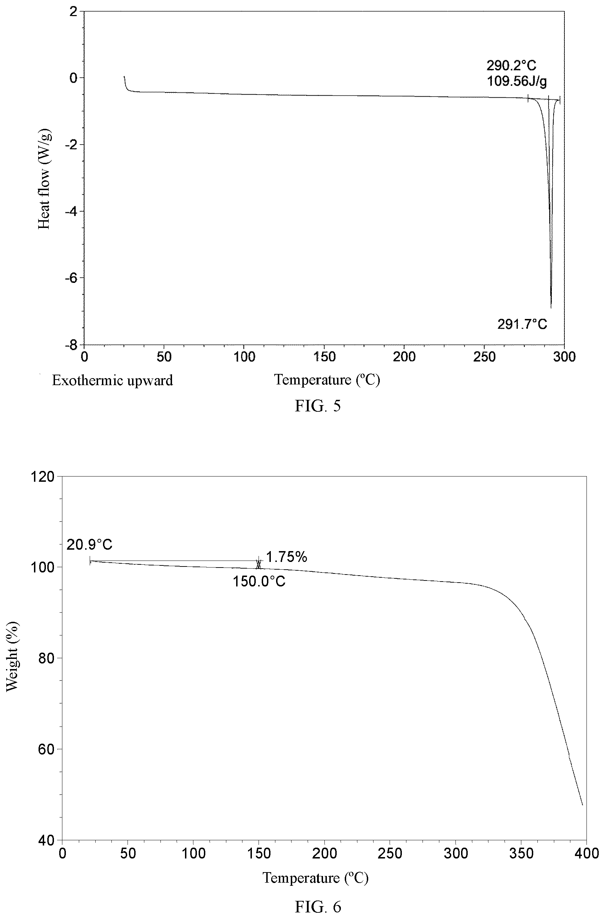

In some embodiments of the present disclosure, the crystal form B of the solvate has a DSC pattern as shown in FIG. 5.

In some embodiments of the present disclosure, the crystal form B of the solvate has a thermogravimetric analysis (TGA) curve with a weight loss of 1.75% at 150°C±3.0°C.

In some embodiments of the present disclosure, the crystal form B of the solvate has a TGA pattern as shown in FIG. 6.

In some embodiments of the present disclosure, the crystal form B of compound 1 may exist in the form of a solvate crystal.

The present disclosure further provides a method for preparing the crystal form B of compound 1, comprising a step of precipitating the crystal form A of compound 1 in methyl tert-butyl ether. Further, a step of separating is included.

In some embodiments of the present disclosure, the crystal form B of compound 1 according to the present disclosure is prepared by the following method:
(1) dissolving compound 1 in *N,N-*dimethylacetamide under sonication;
(2) adding methyl tert-butyl ether after filtration;
(3) stirring at a low temperature for 48 hours and returning to room temperature;
(4) filtering.

In some embodiments of the present disclosure, the low temperature in the step (3) is preferably 5°C.

The present disclosure further provides a crystal form of compound I for use in a method for treating coronavirus infection, comprising administering to an individual in need thereof a therapeutically effective amount of the crystal form A or B of compound 1 according to the present disclosure.

The present disclosure further provides a use of the crystal form A or B of compound 1 in the manufacture of a medicament for treating a disease associated with androgen receptor proteolysis targeting chimera.

In some embodiments of the present disclosure, the disease associated with androgen receptor proteolysis targeting chimera is prostate cancer.

### Technical effect

The crystal forms A and B of compound 1 according to the present disclosure have stable properties, with low hygroscopicity and good prospects for drug development.

### Definition and description

Unless otherwise specified, the following terms and phrases when used herein have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

For any given crystalline form, the preferred orientation resulting from factors such as crystalline morphology can lead to changes in the relative intensities of the diffraction peaks, which is well known in the field of crystallography. Wherever preferred orientation effects are present, the peak intensities may vary, but the diffraction peak positions for the crystal form remain unchanged. Additionally, for any given crystal form, slight errors in peak positions may occur, which is also well known in the field of crystallography. For example, variations in temperature during sample analysis, sample movement, or instrument calibration can lead to shifts in peak positions, resulting in measurement errors of 2θ values, which can sometimes be around ±0.2 degrees. Therefore, it is well understood by those skilled in the art that such errors should be taken into account when determining each crystalline structure.

DSC measures the transition temperature of a crystal when it absorbs or releases heat due to changes in its crystalline structure or crystalline melting. For the same crystal form of the same compound, in consecutive analyses, the error in thermal transition temperature and melting point is typically within about 5°C or 3°C. When we refer to a compound having a given DSC peak or melting point, it refers to the DSC peak or melting point ±5°C or ±3°C. DSC provides an auxiliary method for distinguishing different crystal forms. Different crystalline forms can be identified based on their distinct transition temperature characteristics. It should be noted that for mixtures, the DSC peaks or melting points may vary over a wider range. Furthermore, since decomposition often occurs during the melting of a substance, the melting temperature is related to the heating rate.

For the same crystal form, the weight loss temperature in TGA may vary depending on factors such as the measurement instrument and measurement methods/conditions. For any specific crystal form, there may be an error in the weight loss temperature, which can be about ±5°C or about ±3°C.

It should be noted that during the preparation of crystal forms of a drug, it is difficult to avoid the formation of solvates when drug molecules come into contact with solvent molecules, due to external conditions and internal factors that cause solvent molecules to co-crystallize with compound molecules and remain in the solid substance. These solvates specifically include stoichiometric solvates and non-stoichiometric solvates. The solvates described are all included within the scope of the present disclosure.

The term "pharmaceutically acceptable excipient" refers to an inert substance that is administered along with the active ingredient to facilitate its administration, including, but not limited to, any glidant, sweetener, diluent, preservative, dye/colorant, flavor enhancer, surfactant, wetting agent, dispersant, disintegrant, suspending agent, stabilizer, isotonic agent, solvent, or emulsifier approved by the National Medical Products Administration (NMPA) as acceptable for use in humans or animals (e.g., livestocks).

The term "crystalline composition" refers to a mixture consisting of the crystal form of compound 1 of the present disclosure along with other crystal forms or amorphous forms of the compound, or other impurities. For example, the crystalline composition of crystal form A of compound 1 contains not only crystal form A of compound 1, but also other crystal forms or amorphous forms of compound 1 or other impurities.

The term "pharmaceutical composition" refers to a mixture of one or more compounds of the present disclosure or salts thereof with a pharmaceutically acceptable excipient. The purpose of the pharmaceutical composition is to facilitate the administration of the compound of the present disclosure to an organism.

The therapeutic dosage of the compound of the present disclosure may be determined based on, for example, the specific use of the treatment, the mode of administration of the compound, the health and condition of the patient, and the judgment of the prescribing physician. The proportion or concentration of the compound of the present disclosure in the pharmaceutical composition may not be fixed and depends on a variety of factors including dosage, chemical properties (*e.g.,* hydrophobicity), and route of administration.

The term "treatment" refers to the administration of the compound or formulation of the present disclosure to ameliorate or eliminate a disease or one or more symptoms associated with the disease, and includes:
(i) inhibiting a disease or disease state, *i.e.,* arresting its development;
(ii) alleviating a disease or disease state, *i.e.,* causing its regression.

The term "therapeutically effective amount" refers to an amount of the compound of the present disclosure for (i) treating a specific disease, condition, or disorder; (ii) alleviating, ameliorating, or eliminating one or more symptoms of a specific disease, condition, or disorder, or (iii) preventing or delaying the onset of one or more symptoms of a specific disease, condition, or disorder described herein. The amount of the compound of the present disclosure constituting the "therapeutically effective amount" varies depending on the compound, the disease state and its severity, the mode of administration, and the age of the mammal to be treated, but can be routinely determined by those skilled in the art based on their knowledge and the content of the present disclosure.

Unless the context requires otherwise, throughout the specification and claims thereafter, the term "comprise" and English variations thereof, such as "comprises" and "comprising", are to be construed in an open and inclusive sense, *i.e.,* "including, but not limited to".

Reference throughout the specification to "one embodiment" or "an embodiment" or "another embodiment" or "some embodiments" means that at least one embodiment includes specific reference elements, structures, or characteristics described in connection with the embodiment. Accordingly, the phase "in one embodiment" or "in an embodiment" or "in another embodiment" or "in some embodiments" appearing in various places throughout the specification are not necessarily all referring to the same embodiment. In addition, the specific elements, structures, or characteristics may be combined in any suitable manner in one or more embodiments.

It should be understood that the singular forms of articles "a", "an", and "the" used in the present specification and the appended claims include plural forms, unless explicitly specified otherwise. Thus, for example, a reaction that includes "a catalyst" encompasses the use of one catalyst or two or more catalysts. It should also be understood that the term "or" is generally used to mean "and/or", unless explicitly specified otherwise.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art. Preferred embodiments include, but are not limited to, the examples of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

Unless otherwise specified, the absolute configuration of a stereogenic center is represented by a wedged solid bond ( 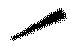 ) and a wedged dashed bond ( 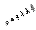 ), and the relative configuration is represented by a straight solid bond ( 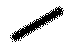 ) and a straight dashed bond ( 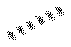 ), for example, trans-1,4-disubstituted cyclohexane is represented by 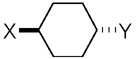 or 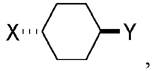 and cis-1,4-disubstituted cyclohexane is represented by 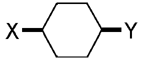 or

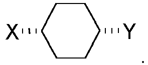

The present disclosure is described in detail by the examples below, but these examples do not imply any restrictions on the present disclosure.

All solvents used in the present disclosure are commercially available and can be used without further purification.

The compounds of the present disclosure are named according to the conventional naming principles in the art or by ChemDraw^{®} software, and the commercially available compounds use the supplier catalog names.

The present disclosure uses the following abbreviations: Boc represents *tert-*butoxycarbonyl; THF represents tetrahydrofuran; DMSO represents dimethyl sulfoxide; EA or EtOAc represents ethyl acetate; MeOH represents methanol; IPA represents isopropanol; ACN represents acetonitrile; DMAC represents N,N-dimethylacetamide; RT represents room temperature.

### Instruments and analytical methods

### 1.1 X-ray powder diffraction (X-ray powder diffractometer, XRPD) method of the present disclosure

Instrument model: PANalytical X-ray powder diffractometer
Test method: Approximately 10 to 20 mg of sample is used for XRPD analysis.

The detailed XRPD parameters are as follows:
Light tube: Cu, kα, (λ = 540598Å, λ = 1.544426Å).
Light tube voltage: 40 kV, light tube current: 40 mA
Divergence slit: 1/8°
Scanning mode: Continuous
Scanning range (°2Theta): 3-40
Time per step (s): 46.7
Step size: 0.0263 s
Test duration: about 5 min

### 1.2 Differential scanning calorimetry (differential scanning calorimeter, DSC) method of the present disclosure

Instrument model: TA 2500 differential scanning calorimeter
Test method: A sample (about 1 mg) is placed in a DSC aluminum pan for testing. The sample is heated from 30°C (room temperature) to 300°C (or 350°C) at a heating rate of 10°C/min under 50 mL/min nitrogen conditions.

### 1.3 Thermogravimetric analysis (thermal gravimetric analyzer, TGA) method of the present disclosure

Instrument model: TA 5500 thermogravimetric analyzer
Test method: A sample (2 to 5 mg) is placed in a TGA platinum pan for testing. The sample is heated from room temperature to 350°C or until a weight loss of 20% at a heating rate of 10°C/min under 25 mL/min nitrogen conditions.

Alternatively:
TGA 550 thermogravimetric analyzer
Test method: A sample (5 to 10 mg) is placed in a TGA platinum pan with an aluminum tray for testing. The sample is heated from room temperature to 300°C at a heating rate of 10°C/min under 60 mL/min nitrogen conditions.

### 1.4 Dynamic vapor sorption (DVS) method of the present disclosure

Instrument model: SEM Advantage-1 dynamic vapor sorption analyzer
Test conditions: A sample (10 to 20 mg) is placed in a DVS sample disk for testing.
The detailed DVS parameters are as follows:
Temperature: 25°C
Equilibrium: dm/dt = 0.01%/min (minimum: 10 min, maximum: 180 min)
Drying: drying for 120 min under 0% RH
RH (%) test gradient: 10%
RH (%) test gradient range: 0% to 95% to 0%.

### BRIEF DESCRIPTION OF THE DRAWINGS

FIG. 1 is the XRPD pattern of crystal form A of compound 1 using Cu-Kα radiation.
FIG. 2 is the DSC pattern of crystal form A of compound 1.
FIG. 3 is the TGA pattern of crystal form A of compound 1.
FIG. 4 is the XRPD pattern of crystal form B of compound 1 using Cu-Kα radiation.
FIG. 5 is the DSC pattern of crystal form B of compound 1.
FIG. 6 is the TGA pattern of crystal form B of compound 1.
FIG. 7 is the DVS pattern of crystal form A of compound 1.
FIG. 8 is the DVS pattern of crystal form B of compound 1.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed. For those skilled in the art, it is obvious to make various modifications and improvements to the specific examples of the present disclosure without departing from the spirit and scope of the present disclosure which is defined in the appended claims.

### Example 1: Preparation of crystal form A of compound 1

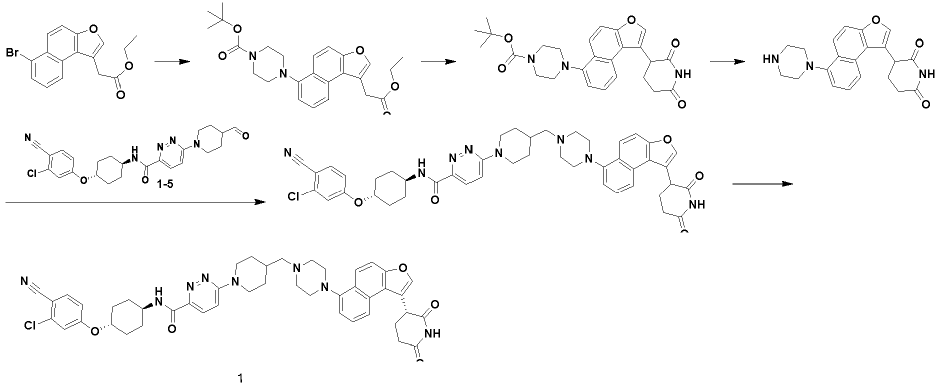

### Step 1: Synthesis of compound 1-2

Under nitrogen atmosphere at room temperature, compound 1-1 (27.86 g, 83.63 mmol), N-Boc-piperazine (23.36 g, 125.44 mmol), tris(dibenzylideneacetone)dipalladium (7.66 g, 8.36 mmol), 2-di-tert-butylphosphino-2',4',6'-triisopropylbiphenyl (7.10 g, 16.73 mmol), and potassium phosphate (53.25 g, 250.88 mmol) were added to H₂O (50 mL) and 1,4-dioxane (500 mL). The reaction mixture was slowly heated to 100°C and stirred for 12 hours. After the reaction was completed, the reaction mixture was poured into water (500 mL) and extracted with ethyl acetate (500 mL × 2). The organic phases were combined, washed with saturated brine (500 mL × 2), dried over anhydrous sodium sulfate, and filtered. The filtrate was concentrated under reduced pressure. The resulting residue was separated by column chromatography (eluent: petroleum ether/ethyl acetate = 1/0 to 93/7, v/v) to obtain compound **1-2.** ¹H NMR (400 MHz, CDCl₃) δ: 8.21 (d, *J* = 9.3 Hz, 1H), 7.97 (d, *J* = 8.3 Hz, 1H), 7.75 (s, 1H), 7.65 (d, *J* = 9.3 Hz, 1H), 7.51 (t, *J =* 7.9 Hz, 1H), 7.12 (d, *J =* 7.3 Hz, 1H), 4.26 - 4.06 (m, 6H), 3.44 - 2.70 (m, 6H), 1.52 (s, 9H), 1.27 (t, *J* = 7.1 Hz, 3H).

### Step 2: Synthesis of compound 1-3

Under nitrogen atmosphere at room temperature, compound 1-2 (25 g, 57.01 mmol), acrylamide (6.08 g, 85.52 mmol, 5.90 mL), and potassium tert-butoxide (9.60 g, 85.52 mmol) were dissolved in N,N-dimethylformamide (300 mL). The reaction mixture was stirred at 0 to 5°C for 2 hours. After the reaction was completed, the reaction mixture was poured into a saturated ammonium chloride solution (400 mL), and filtered. The filter cake was washed with water (250 mL) and rotary evaporated to dryness. The resulting crude product was purified by slurrying in methanol (100 mL, 25°C, 12 hours) to obtain compound **1-3.** ¹H NMR (400 MHz, DMSO*_d₆*) δ: 10.95 (s, 1H), 8.20 (d, *J =* 9.2 Hz, 1H), 8.00 (s, 1H), 7.90 (m, 1H), 7.78 (d, *J =* 9.2 Hz, 1H), 7.52 (m, 1H), 7.20 (d, *J =* 7.6 Hz, 1H), 4.66 (m, 1H), 3.29 - 3.12 (m, 4H), 3.03 - 2.57 (m, 5H), 2.45 - 2.21 (m, 3H), 1.45 (s, 9H).

### Step 3: Synthesis of hydrochloride of compound 1-4

Compound **1-3** (21.5 g, 46.38 mmol) was dissolved in ethyl acetate (500 mL) at room temperature, and then a solution of hydrochloric acid in ethyl acetate (4 M, 250 mL) was added thereto. The reaction mixture was stirred at room temperature for 48 hours. After the reaction was completed, the reaction mixture was concentrated under reduced pressure to obtain a crude product, which was purified by slurrying in ethyl acetate (30 mL) at room temperature to obtain a hydrochloride of compound **1-4.** ¹H NMR (400 MHz, DMSO*_d₆*) δ: 10.95 (s, 1H), 9.54 (br, 1H), 8.19 (d, *J =* 9.2 Hz, 1H), 8.05 - 7.89 (m, 2H), 7.80 (d, *J =* 9.2 Hz, 1H), 7.55 (m, 1H), 7.22 (d, *J* = 7.6 Hz, 1H), 4.74 - 4.62 (m, 1H), 3.44 - 3.19 (m, 8H), 2.85 - 2.95 (m, 1H), 2.60 - 2.66 (m, 1H), 2.47 - 2.36 (m, 1H), 2.20 - 2.30 (m, 1H).

### Step 4: Synthesis of compound 1-6

Under nitrogen atmosphere at room temperature, the hydrochloride of compound **1-4** (16.35 g, 40.89 mmol), compound **1-5** (16.0 g, 34.12 mmol), and sodium triacetoxyborohydride (10.83 g, 51.11 mmol) were dissolved in dichloromethane (1500 mL). The reaction mixture was stirred at room temperature for 48 hours, and sodium acetate (5.59 g, 68.15 mmol) was added thereto. The reaction mixture was stirred for another 24 hours. After the reaction was completed, the reaction mixture was quenched with water (500 mL). The resulting phases were separated. The organic phase was washed with saturated brine (500 mL × 2), dried over anhydrous sodium sulfate, and filtered. The filtrate was concentrated under reduced pressure. The resulting residue was separated by column chromatography (eluent: ethyl acetate/methanol = 95/5, v/v). The crude product was slurried with ethyl acetate (50 mL) at room temperature to obtain compound **1-6.** MS-ESI *m*/*z:* 815.2 [M+H]⁺. ¹H NMR (400 MHz, DMSO*_d₆*) δ: 10.95 (s, 1H), 8.59 (d, *J =* 8.4 Hz, 1H), 8.16 (d, *J =* 9.2 Hz, 1H), 7.99 (s, 1H), 7.92 - 7.73 (m, 4H), 7.53 - 7.40 (m, 1H), 7.43 - 7.29 (m, 2H), 7.24 - 7.09 (m, 2H), 4.72 - 4.60 (m, 1H), 4.59 - 4.45 (m, 3H), 3.93 - 3.80 (m, 1H), 3.24 - 2.96 (m, 6H), 2.95 - 2.82 (m, 2H), 2.76 - 2.58 (m, 3H), 2.48 - 2.18 (m, 5H), 2.16 - 2.06 (m, 2H), 1.99 - 1.79 (m, 5H), 1.72 - 1.44 (m, 4H), 1.25 - 1.07 (m, 2H).

### Step 5: Chiral HPLC resolution

Compound **1-6** was subjected to chiral HPLC resolution (column: DAICEL CHIRALPAK IE (250 mm * 30 mm, 10 µm); mobile phase: A (IPA), B (ACN), B%: 50% to 100%; flow rate: 80 mL/min) to obtain a crystal form A of compound **1** (whose XRPD pattern is shown in FIG. 1).

Analysis method: chromatographic column: Chiralpak IA 100 * 4.6 mm, 3 µm; mobile phase: A: *n*-hexane (0.1% diethylamine); B: isopropanol: acetonitrile = 2:1, A:B = 40:60; column temperature: 35°C; wavelength: 220 nm.

Compound **1** (first major peak, retention time: 4.857 min): ¹H NMR (400 MHz, DMSO*_d₆*) δ: 10.95 (s, 1H), 8.60 (d, *J* = 8.4 Hz, 1H), 8.16 (d, *J* = 9.2 Hz, 1H), 8.00 (s, 1H), 7.91 - 7.74 (m, 4H), 7.53 - 7.40 (m, 1H), 7.43 - 7.30 (m, 2H), 7.23 - 7.09 (m, 2H), 4.72 - 4.60 (m, 1H), 4.60 - 4.45 (m, 3H), 3.94 - 3.80 (m, 1H), 3.24 - 2.96 (m, 6H), 2.95 - 2.79 (m, 2H), 2.76 - 2.58 (m, 3H), 2.45 - 2.18 (m, 5H), 2.16 - 2.06 (m, 2H), 2.03 - 1.82 (m, 5H), 1.73 - 1.44 (m, 4H), 1.24 - 1.09 (m, 2H).

### Example 2: Preparation of crystal form B of compound 1

At room temperature, the crystal form A of compound **1** (3.35 g) was dissolved in DMAC (100 mL) with the assistance of sonication to aid dissolution. After filtration, methyl tert-butyl ether (1000 mL) was added thereto, and the mixture was cooled to 5°C and stirred for 48 hours, resulting in solid precipitation. The mixture was allowed to return to room temperature, and filtered. The filter cake obtained was a crystal form B of compound 1 (whose XRPD pattern is shown in FIG. 4).

Analysis method: chromatographic column: Chiralpak IA 100 * 4.6 mm, 3 µm; mobile phase: A: *n*-hexane (0.1% diethylamine); B: isopropanol: acetonitrile = 2:1, A:B = 40:60; column temperature: 35°C; wavelength: 220 nm; retention time: 4.856 min; MS-ESI *m*/*z:* 815.3 [M+H]⁺.

### Experimental example 1: Hygroscopicity study of crystal form A of compound 1

### Experimental materials:

SEM Advantage-1 dynamic vapor sorption analyzer

### Experimental methods:

10 to 20 mg of the crystal form A of compound 1 was taken and placed in a DVS sample disk for testing.

### Experimental results:

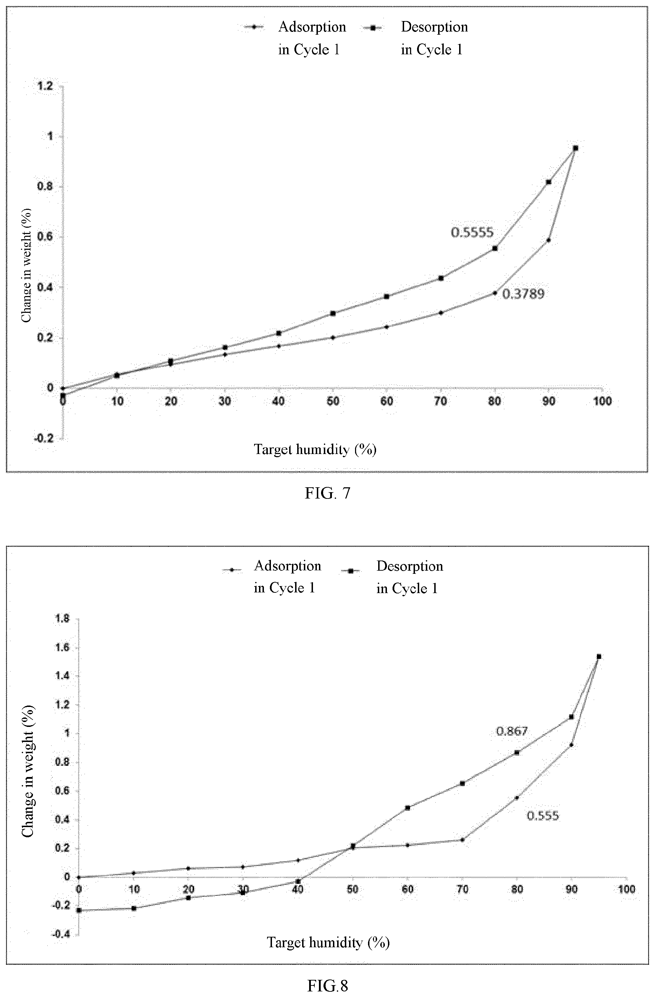

The DVS pattern of the crystal form A of compound 1 is shown in FIG. 7.

### Experimental conclusion:

The hygroscopic weight gain of the crystal form A of compound **1** was 0.3789% at 25°C and 80% RH, indicating slight hygroscopicity. The crystal form remained unchanged before and after the DVS test.

### Experimental example 2: Hygroscopicity study of crystal form B of compound 1

### Experimental materials:

SEM Advantage-1 dynamic vapor sorption analyzer

### Experimental methods:

10 to 20 mg of the crystal form B of compound **1** was taken and placed in a DVS sample disk for testing.

### Experimental results:

The DVS pattern of the crystal form B of compound **1** is shown in FIG. 8, △W = 0.555%.

### Experimental conclusion:

The hygroscopic weight gain of the crystal form B of compound **1** was 0.555% at 25°C and 80% RH, indicating slight hygroscopicity. The crystal form remained unchanged before and after the DVS test.

### Experimental example 3: Interconversion relationship of crystal forms A and B of compound 1 in different solvents

The interconversion relationship between the two anhydrous crystal forms (crystal form A and crystal form B) was studied using a suspension competition assay.

Approximately 20 mg of the crystal form A sample was weighed and suspended in 1 mL of solvent. After stirring at the corresponding temperature, the mixture was filtered, and the filtrate was transferred to HPLC vials containing both the crystal forms A and B. The suspension was stirred at the corresponding temperature, followed by centrifugation. The wet sample was analyzed by XRPD. The results are summarized in Table 3.

**Table 3. Summary of suspension competition results between crystal forms A and B**

| **Experiment No.** | **Starting material** | **Solvent** | **Temperature** | **Time** | **Results** |
|---|---|---|---|---|---|
| 1 | | THF | RT | Day 5 | Crystal form B |
| 2 | Crystal forms A+B | EtOAc | | Day 5 | Crystal form B |
| 3 | | THF | 50°C | Day 5 | Crystal form B |
| 4 | | EtOAc | | Day 5 | Crystal form B |

Experimental conclusion: The results indicate that the crystal form B was obtained in all solvent systems at different temperatures.

### Experimental example 4: Solid stability test of crystal form B of compound 1 under accelerated conditions

In accordance with the "Guidelines for Stability Testing of APIs and Formulations" (Chinese Pharmacopoeia 2020 Edition, Volume IV, General Principles 0512), the stability of the crystal form B of compound **1** was evaluated under accelerated conditions of high temperature and high humidity (40°C/75% relative humidity, sealed).

Approximately 1.2 g of the crystal form B of compound **1** was weighed and placed in a double-layer low-density polyethylene (LDPE) bag. Each layer of the LDPE bag was individually sealed tightly, and placed in an aluminum foil bag, which was then heat-sealed. Samples were taken for analysis at the 1st, 2nd, and 3rd months, and the results were compared to the initial test results on day 0. The experimental results are shown in Table 4 below.

**Table 4. Solid stability test results of crystal form B of compound 1 under accelerated conditions (40°C/75% relative humidity, sealed)**

| Test conditions | Sampling time point | Appearance | Content | Total impurities | Isomer | Crystal form (XRPD) |
|---|---|---|---|---|---|---|
| - | Day 0 | White powder | 94.0% | 0.83% | 1.46% | Crystal form B |
| 40°C/75% relative humidity, sealed | 1 month | White powder | 94.5% | 0.91% | 1.32% | Not tested |
| | 2 months | White powder | 94.7% | 0.84% | 1.35% | Not tested |
| | 3 months | White powder | 96.0% | 0.78% | 1.42% | Crystal form B |

Conclusion: The crystal form B of compound **1** demonstrates good stability under accelerated conditions of 40°C/75% relative humidity.

### Experimental example 5: Solid stability test of crystal form B of compound 1 under long-term conditions

In accordance with the "Guidelines for Stability Testing of APIs and Formulations" (Chinese Pharmacopoeia 2020 Edition, Volume IV, General Principles 0512), the stability of the crystal form B of compound **1** was evaluated under long-term conditions (25°C/60% relative humidity, sealed).

Approximately 1.2 g of the crystal form B of compound **1** was weighed and placed in a double-layer low-density polyethylene (LDPE) bag. Each layer of the LDPE bag was individually sealed tightly, and placed in an aluminum foil bag, which was then heat-sealed. Samples were taken for analysis at the 3rd month, and the results were compared to the initial test results on day 0. The experimental results are shown in Table 5 below.

**Table 5. Solid stability test results of crystal form B of compound 1 under long-term conditions (25°C/60% relative humidity, sealed)**

| Test conditions | Sampling time point | Appearance | Content | Total impurities | Isomer | Crystal form (XRPD) |
|---|---|---|---|---|---|---|
| - | Day 0 | White powder | 94.0% | 0.83% | 1.46% | Crystal form B |
| 25°C/60% relative humidity, sealed | 3 months | White powder | 96.1% | 0.74% | 1.40% | Crystal form B |

Conclusion: The crystal form B of compound 1 demonstrates good stability under long-term conditions of 25°C/60% relative humidity.

### Bioassay

Test example 1: Study of the anti-proliferative effect of compound 1 in LNCap cells with AR F877L mutation by *in vitro* cell viability assay

Objective: This experiment aims to investigate the anti-proliferative effect of compound 1 by evaluating its impact on *in vitro* cell viability in LNCap cells with the AR F877L mutation.

### Experimental materials:

### 1. Cell line and culture method

**Table 6. Cell line and culture method**

| Cell line | Tumor type | Growth characteristics | Culture method |
|---|---|---|---|
| LNCap with AR F877L mutation | Prostate cancer cell | Adherent growth | 1640+10% FBS+1% AA |

### 2. Culture media and reagents

**Table 7. Culture media and reagents**

| Culture media and reagents | Manufacturers | Cat. No. |
|---|---|---|
| RPMI 1640 | GIBCO | 22400-089 |
| PBS | Cytiva | SH30256.01 |
| FBS | Hyclone | SH30084.03 |
| Antibiotic-antimycotic | GIBCO | 15240-062 |
| 0.25% Trypsin | GIBCO | 25200072 |
| DMSO | SIGMA | D2650 |

### 3. Experimental equipment

1) Greiner CELLSTAR 96-well plate, flat-bottom black plate (with lid and clear bottom), #655090
2) Promega CellTiter-Glo luminescent cell viability assay kit (Promega-G7573).
3) 2104 EnVision plate reader, PerkinElmer.

### Experimental method:

### 1. Cell culture:

The tumor cell lines were cultured under the conditions specified in Table 6 in an incubator at 37°C with 5% CO₂. Cells were routinely passaged, and the cells in the logarithmic growth phase were used for plating.

### 2. Cell plating:

1) Cells were stained with trypan blue and viable cells were counted.
2) The cell concentration was adjusted to an appropriate concentration with a density of 5000 cells/well.
3) 90 µL of cell suspension per well was added to a culture plate, and cell-free culture medium was added to the blank control well.
4) The culture plate was cultured overnight in a 37°C, 5% CO₂ incubator with 100% relative humidity.

### 3. Preparation of compound storage plate and viability assay

1) Preparation of 400X compound storage plate: The compound was serially diluted with DMSO from the highest concentration to the lowest concentration.
2) Preparation of 10X compound working solution: To a V-bottom 96-well plate was added 78 µL of cell culture medium, and 2 µL of compound was pipetted from a 400X compound storage plate and added to the cell culture medium of the 96-well plate. 2 µL of DMSO was added to vehicle control and blank control. After the compound or DMSO was added, blowing with a pipette and mixing well were carried out.
3) Administration: 10 µL of 10X compound working solution was added to the cell culture plate. To vehicle control and blank control was added 10 µL of DMSO-cell culture medium mixture. The final concentration of DMSO was 0.25%.
4) The 96-well plate was returned to the incubator and cultured for 144 hours.
5) CellTiter-Glo buffer was thawed and brought to room temperature.
6) CellTiter-Glo substrate was also brought to room temperature.
7) CellTiter-Glo buffer was added to a bottle of CellTiter-Glo substrate to dissolve the substrate, thereby preparing CellTiter-Glo working solution.
8) The mixture was slowly vortexed and shaken to be fully dissolved.
9) The cell culture plate was removed and allowed to equilibrate to room temperature for 30 minutes.
10) 50 µL (equal to half the volume of cell culture medium in each well) of CellTiter-Glo working solution was added to each well. The cell plate was wrapped in aluminum foil to be protected from light.
11) The culture plate was shaken on an orbital shaker for 2 minutes to induce cell lysis.
12) The plate was then left at room temperature for 10 minutes to stabilize the luminescent signal.
13) The luminescent signal was detected on a 2104 EnVision plate reader.

### 4. Data analysis

The inhibition rate (IR) of the test compound was calculated using the following formula: IR (%) = (RLU vehicle control - RLU compound) / (RLU vehicle control - RLU blank control) × 100%. The inhibition rates of compounds at different concentrations were calculated in Excel, and then GraphPad Prism software was used to plot inhibition curves and calculate relevant parameters, including minimum inhibition rate, maximum inhibition rate, and IC₅₀.

### 5. Experimental results

**Table 8. Anti-proliferative parameters from cell viability assay**

| Compound | IC₅₀ in LNCap cells with AR F877L mutation (nM) |
|---|---|
| Compound 1 | 167 |

Conclusion: The compound 1 of the present disclosure exhibits excellent inhibitory effect on cell proliferation in LNCap cells with AR F877L mutation.

## Claims

1. A crystal form A of compound **1,** 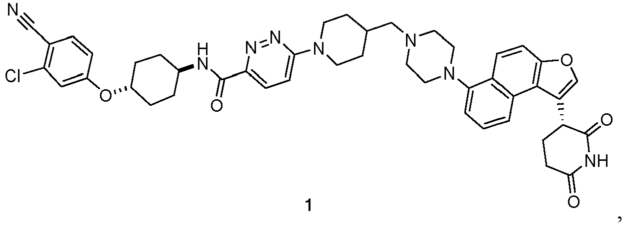 wherein the crystal form A has an X-ray powder diffraction pattern comprising characteristic diffraction peaks at the following 2θ angles: 17.521±0.200°, 19.495±0.200°, 20.531±0.200°, 20.956±0.200°, and 22.271±0.200°.

2. The crystal form A of compound 1 according to claim 1, wherein the X-ray powder diffraction pattern for the crystal form A, represented by 2θ angles, comprises at least 5, 6, 7, or 8 diffraction peaks selected from the following: 9.530±0.200°, 12.135±0.200°, 12.784±0.200°, 17.521±0.200°, 19.495±0.200°, 20.531±0.200°, 20.956±0.200°, and 22.271±0.200°.

3. The crystal form A of compound 1 according to claim 1, wherein the X-ray powder diffraction pattern for the crystal form A comprises characteristic diffraction peaks at the following 2θ angles: 9.530±0.200°, 12.135±0.200°, 12.784±0.200°, 17.521±0.200°, 19.495±0.200°, 20.531±0.200°, 20.956±0.200°, and 22.271±0.200°;
preferably, the X-ray powder diffraction pattern for the crystal form A comprises characteristic diffraction peaks at the following 2θ angles: 9.530±0.200°, 12.135±0.200°, 12.784±0.200°, 16.622±0.200°, 16.973±0.200°, 17.521±0.200°, 17.858±0.200°, 18.617±0.200°, 19.495±0.200°, 20.531±0.200°, 20.956±0.200°, 22.271±0.200°, and 22.815±0.200°.

4. The crystal form A of compound 1 according to claim 1, wherein the X-ray powder diffraction pattern for the crystal form A, represented by 2θ angles, comprises at least 10, 11, 12, or 13 diffraction peaks selected from the following: 9.530±0.200°, 12.135±0.200°, 12.784±0.200°, 16.622±0.200°, 16.973±0.200°, 17.521±0.200°, 17.858±0.200°, 18.617±0.200°, 19.495±0.200°, 20.531±0.200°, 20.956±0.200°, 22.271±0.200°, and 22.815±0.200°.

5. The crystal form A of compound 1 according to claim 1, wherein the X-ray powder diffraction pattern for the crystal form A comprises characteristic diffraction peaks at the following 2θ angles: 4.358°, 9.530°, 12.135°, 12.784°, 14.166°, 15.597°, 16.622°, 16.973°, 17.521°, 17.858°, 18.617°, 19.495°, 20.531°, 20.956°, 22.271°, 22.815°, 24.711°, 25.955°, 26.976°, 27.682°, 28.728°, 30.119°, 31.329°, 33.598°, and 35.500°.

6. The crystal form A of compound 1 according to claim 1, wherein the X-ray powder diffraction pattern for the crystal form A is as shown in FIG. 1;
or, the differential scanning calorimetry curve for the crystal form A is as shown in FIG. 2;
or, the thermogravimetric analysis curve for the crystal form A is as shown in FIG. 3.

7. The crystal form A of compound 1 according to claim 1, wherein the crystal form A has a differential scanning calorimetry curve comprising an endothermic peak with an onset at 279.6°C±3.0°C;
or, the crystal form A has a thermogravimetric analysis curve with a weight loss of 0.59% at 150.0°C±3°C.

8. A crystal form B of compound **1,** 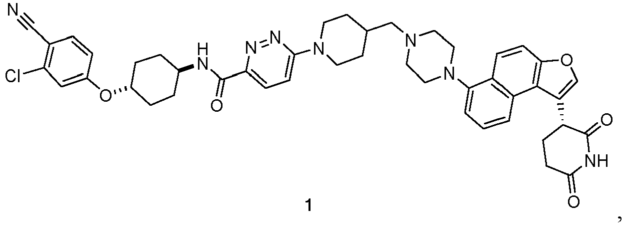 wherein the crystal form B has an X-ray powder diffraction pattern comprising characteristic diffraction peaks at the following 2θ angles: 10.655±0.200°, 11.988±0.200°, 16.055±0.200°, 18.356±0.200°, and 20.083±0.200°.

9. The crystal form B of compound 1 according to claim 8, wherein the X-ray powder diffraction pattern for the crystal form B, represented by 2θ angles, comprises at least 5, 6, 7, or 8 diffraction peaks selected from the following: 16.039±0.200°, 16.923±0.200°, 18.394±0.200°, 19.070±0.200°, 19.445±0.200°, 20.915±0.200°, 21.271±0.200°, and 21.599±0.200°.

10. The crystal form B of compound 1 according to claim 8, wherein the X-ray powder diffraction pattern for the crystal form B comprises characteristic diffraction peaks at the following 2θ angles: 16.039±0.200°, 16.923±0.200°, 18.394±0.200°, 19.070±0.200°, 19.445±0.200°, 20.915±0.200°, 21.271±0.200°, and 21.599±0.200°;
preferably, the X-ray powder diffraction pattern for the crystal form B comprises characteristic diffraction peaks at the following 2θ angles: 5.222±0.200°, 10.412±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, 23.951±0.200°, and 25.463±0.200°.

11. The crystal form B of compound 1 according to claim 8, wherein the X-ray powder diffraction pattern for the crystal form B, represented by 2θ angles, comprises at least 10, 11, 12, or 13 diffraction peaks selected from the following: 5.222±0.200°, 10.412±0.200°, 14.668±0.200°, 16.039±0.200°, 16.923±0.200°, 18.394±0.200°, 19.070±0.200°, 19.445±0.200°, 20.915±0.200°, 21.271±0.200°, 21.599±0.200°, 23.951±0.200°, and 25.463±0.200°.

12. The crystal form B of compound 1 according to claim 8, wherein the X-ray powder diffraction pattern for the crystal form B comprises characteristic diffraction peaks at the following 2θ angles: 5.222°, 8.368°, 10.412°, 13.031°, 14.668°, 16.039°, 16.923°, 18.394°, 19.070°, 19.445°, 20.915°, 21.271°, 21.599°, 23.591°, 25.463°, 27.635°, and 31.559°.

13. The crystal form B of compound 1 according to claim 8, wherein the X-ray powder diffraction pattern for the crystal form B is as shown in FIG. 4;
or, the differential scanning calorimetry curve for the crystal form B is as shown in FIG. 5;
or, the thermogravimetric analysis curve for the crystal form B is as shown in FIG. 6.

14. The crystal form B of compound 1 according to claim 8, wherein the crystal form B has a differential scanning calorimetry curve comprising an endothermic peak with a peak value at 290.2°C±3.0°C;
or, the crystal form B has a thermogravimetric analysis curve with a weight loss of 1.75% at 150.0°C±3°C.

15. The crystal form A of compound 1 according to claims 1 to 7 or the crystal form B of compound 1 according to claims 8 to 14 for use in the treatment of a disease associated with androgen receptor proteolysis targeting chimera, wherein the disease associated with androgen receptor proteolysis targeting chimera is preferably prostate cancer.

## Patentansprüche

1. Kristallform A von Verbindung 1, 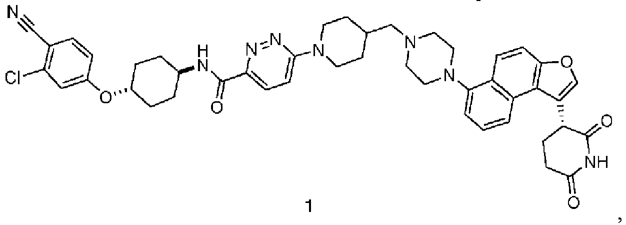 wobei die Kristallform A ein Pulver-Röntgenbeugungsmuster aufweist, das charakteristische Beugungsmaxima bei den folgenden 2θ-Winkeln umfasst: 17,521±0,200°, 19,495±0,200°, 20,531±0,200°, 20,956±0,200°und 22,271±0,200°.

2. Kristallform A von Verbindung 1 nach Anspruch 1, wobei das durch 2θ Winkel repräsentierte Pulver-Röntgenbeugungsmuster für die Kristallform A mindestens 5, 6, 7 oder 8 Beugungsmaxima umfasst, die aus den folgenden ausgewählt sind: 9,530±0,200°, 12,135±0,200°, 12,784±0,200°, 17,521±0,200°, 19,495±0,200°, 20,531±0,200°, 20,956±0,200° und 22,271±0,200°.

3. Kristallform A von Verbindung 1 nach Anspruch 1, wobei das Pulver-Röntgenbeugungsmuster für die Kristallform A charakteristische Beugungsmaxima bei den folgenden 2θ-Winkeln umfasst: 9,530±0,200°, 12,135±0,200°, 12,784±0.200°, 17,521±0,200°, 19,495±0,200°, 20,531±0,200°, 20,956±0,200°und 22,271±0,200°;
vorzugsweise wobei das Pulver-Röntgenbeugungsmuster für die Kristallform A charakteristische Beugungsmaxima bei den folgenden 2θ-Winkeln umfasst: 9,530±0,200°, 12,135±0,200°, 12,784±0,200°, 16,622±0,200°, 16,973±0,200°, 17,521±0,200°, 17,858±0,200°, 18,617±0,200°, 19,495±0,200°, 20,531±0,200°, 20,956±0,200°, 22,271±0,200° und 22,815±0,200°.

4. Kristallform A von Verbindung 1 nach Anspruch 1, wobei das durch 2θ Winkel repräsentierte Pulver-Röntgenbeugungsmuster für die Kristallform A mindestens 10, 11, 12 oder 13 Beugungsmaxima umfasst, die aus den folgenden ausgewählt sind: 9,530±0,200°, 12,135±0,200°, 12,784±0,200°, 16,622±0,200°, 16,973±0,200°, 17,521±0,200°, 17,858±0,200°, 18,617±0,200°, 19,495±0,200°, 20,531±0,200°, 20,956±0,200°, 22,271±0,200° und 22,815±0,200°.

5. Kristallform A von Verbindung 1 nach Anspruch 1, wobei das Pulver-Röntgenbeugungsmuster für die Kristallform A charakteristische Beugungsmaxima bei den folgenden 2θ-Winkeln umfasst: 4,358°, 9,530°, 12,135°, 12,784°, 14,166°, 15,597°, 16,622°, 16,973°, 17,521°, 17,858°, 18,617°, 19,495°, 20,531°, 20,956°, 22,271°, 22,815°, 24,711°, 25,955°, 26,976°, 27,682°, 28,728°, 30,119°, 31,329°, 33,598° und 35,500°.

6. Kristallform A von Verbindung 1 nach Anspruch 1, wobei das Pulver-Röntgenbeugungsmuster für die Kristallform A wie in FIG. 1 dargestellt ist;
oder die Dynamische-Differenzkalorimetrie-Kurve für die Kristallform A wie in FIG. 2 dargestellt ist;
oder die Thermogravimetrie-Kurve für die Kristallform A wie in FIG. 3 dargestellt ist.

7. Kristallform A von Verbindung 1 nach Anspruch 1, wobei die Kristallform A eine Dynamische-Differenzkalorimetrie-Kurve aufweist, die ein endothermes Maximum mit Beginn bei 279,6 °C±3,0 °C umfasst;
oder die Kristallform A eine Thermogravimetrie-Kurve mit einem Gewichtsverlust von 0,59 % bei 150,0 °C±3 °C aufweist.

8. Kristallform B von Verbindung 1, 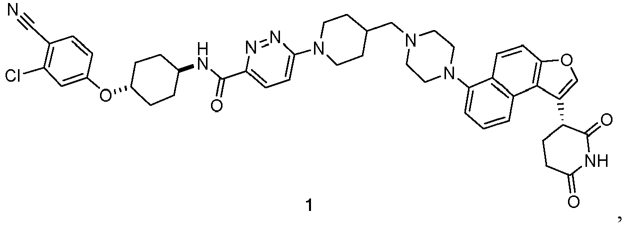 wobei die Kristallform B ein Pulver-Röntgenbeugungsmuster aufweist, das charakteristische Beugungsmaxima bei den folgenden 2θ-Winkeln umfasst: 10,655±0,200°, 11,988±0,200°, 16,055±0,200°, 18,356±0,200°und 20,083±0,200°.

9. Kristallform B von Verbindung 1 nach Anspruch 8, wobei das durch 2θ Winkel repräsentierte Pulver-Röntgenbeugungsmuster für die Kristallform B mindestens 5, 6, 7 oder 8 Beugungsmaxima umfasst, die aus den folgenden ausgewählt sind: 16,039±0,200°, 16,923±0,200°, 18,394±0,200°, 19,070±0,200°, 19,445±0,200°, 20,915±0,200°, 21,271±0,200° und 21,599±0,200°.

10. Kristallform B von Verbindung 1 nach Anspruch 8, wobei das Pulver-Röntgenbeugungsmuster für die Kristallform B charakteristische Beugungsmaxima bei den folgenden 2θ-Winkeln umfasst: 16,039±0,200°, 16,923±0,200°, 18,394±0.200°, 19,070±0,200°, 19,445±0,200°, 20,915±0,200°, 21,271±0,200°und 21,599±0,200°;
vorzugsweise wobei das Pulver-Röntgenbeugungsmuster für die Kristallform B charakteristische Beugungsmaxima bei den folgenden 2θ-Winkeln umfasst: 5,222±0,200°, 10,412±0,200°, 14,668±0,200°, 16,039±0,200°, 16,923±0,200°, 18,394±0,200°, 19,070±0,200°, 19,445±0,200°, 20,915±0,200°, 21,271±0,200°, 21,599±0,200°, 23,951±0,200° und 25,463±0,200°.

11. Kristallform B von Verbindung 1 nach Anspruch 8, wobei das durch 2θ Winkel repräsentierte Pulver-Röntgenbeugungsmuster für die Kristallform B mindestens 10, 11, 12 oder 13 Beugungsmaxima umfasst, die aus den folgenden ausgewählt sind: 5,222±0,200°, 10,412±0,200°, 14,668±0,200°, 16,039±0,200°, 16,923±0,200°, 18,394±0,200°, 19,070±0,200°, 19,445±0,200°, 20,915±0,200°, 21,271±0,200°, 21,599±0,200°, 23,951±0,200° und 25,463±0,200°.

12. Kristallform B von Verbindung 1 nach Anspruch 8, wobei das Pulver-Röntgenbeugungsmuster für die Kristallform B charakteristische Beugungsmaxima bei den folgenden 2θ-Winkeln umfasst: 5,222°, 8,368°, 10,412°, 13,031°, 14,668°, 16,039°, 16,923°, 18,394°, 19,070°, 19,445°, 20,915°, 21,271°, 21,599°, 23,591°, 25,463°, 27,635° und 31,559°.

13. Kristallform B von Verbindung 1 nach Anspruch 8, wobei das Pulver-Röntgenbeugungsmuster für die Kristallform B wie in FIG. 4 dargestellt ist;
oder die Dynamische-Differenzkalorimetrie-Kurve für die Kristallform B wie in FIG. 5 dargestellt ist;
oder die Thermogravimetrie-Kurve für die Kristallform B wie in FIG. 6 dargestellt ist.

14. Kristallform B von Verbindung 1 nach Anspruch 8, wobei die Kristallform B eine Dynamische-Differenzkalorimetrie-Kurve aufweist, die ein endothermes Maximum mit einem Spitzenwert bei 290,2 °C±3,0 °C umfasst;
oder die Kristallform B eine Thermogravimetrie-Kurve mit einem Gewichtsverlust von 1,75 % bei 150,0 °C±3 °C aufweist.

15. Kristallform A von Verbindung 1 nach Anspruch 1 bis 7 oder Kristallform B von Verbindung 1 nach Anspruch 8 bis 14 zur Verwendung bei der Behandlung einer Krankheit, die mit Androgenrezeptor-PROTAC (Proteolysis Targeting Chimera) assoziiert ist, wobei es sich bei der Krankheit, die mit Androgenrezeptor-PROTAC assoziiert ist, bevorzugt um Prostatakrebs handelt.

## Revendications

1. Forme cristalline A du composé 1, 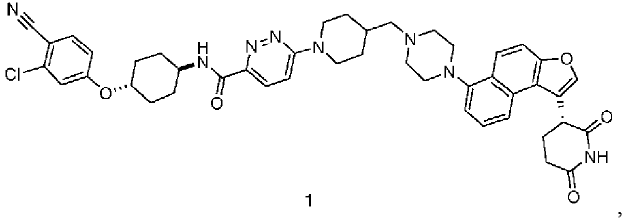 dans laquelle la forme cristalline A présente un diagramme de diffraction des rayons X sur poudre comprenant des pics de diffraction caractéristiques aux angles 2θ suivants : 17,521 ± 0,200°, 19,495 ± 0,200°, 20,531 ± 0,200°, 20,956 ± 0,200°, et 22,271 ± 0,200°.

2. Forme cristalline A du composé 1 selon la revendication 1, dans laquelle le diagramme de diffraction des rayons X sur poudre pour la forme cristalline A, représenté par des angles 2θ, comprend au moins 5, 6, 7, ou 8 pics de diffraction choisis parmi les suivants : 9,530 ± 0,200°, 12,135 ± 0,200°, 12,784 ± 0,200°, 17,521 ± 0,200°, 19,495 ± 0,200°, 20,531 ± 0,200°, 20,956 ± 0,200°, et 22,271 ± 0,200°.

3. Forme cristalline A du composé 1 selon la revendication 1, dans laquelle le diagramme de diffraction des rayons X sur poudre pour la forme cristalline A comprend des pics de diffraction caractéristiques aux angles 2θ suivants : 9,530 ± 0,200°, 12,135 ± 0,200°, 12,784 ± 0,200°, 17,521 ± 0,200°, 19,495 ± 0,200°, 20,531 ± 0,200°, 20,956 ± 0,200°, et 22,271 ± 0,200°;
de préférence, le diagramme de diffraction des rayons X sur poudre pour la forme cristalline A comporte des pics de diffraction caractéristiques aux angles 2θ suivants : 9,530 ± 0,200°, 12,135 ± 0,200°, 12,784 ± 0,200°, 16,622 ± 0,200°, 16,973 ± 0,200°, 17,521 ± 0,200°, 17,858 ± 0,200°, 18,617 ± 0,200°, 19,495 ± 0,200°, 20,531 ± 0,200°, 20,956 ± 0,200°, 22,271 ± 0,200°, et 22,815 ± 0,200°.

4. Forme cristalline A du composé 1 selon la revendication 1, dans laquelle le diagramme de diffraction des rayons X sur poudre pour la forme cristalline A, représenté par des angles 2θ, comprend au moins 10, 11, 12, ou 13 pics de diffraction choisis parmi les suivants : 9,530 ± 0,200°, 12,135 ± 0,200°, 12,784 ± 0,200°, 16,622 ± 0,200°, 16,973 ± 0,200°, 17,521 ± 0,200°, 17,858 ± 0,200°, 18,617 ± 0,200°, 19,495 ± 0,200°, 20,531 ± 0,200°, 20,956 ± 0,200°, 22,271 ± 0,200°, et 22,815 ± 0,200°.

5. Forme cristalline A du composé 1 selon la revendication 1, dans laquelle le diagramme de diffraction des rayons X sur poudre pour la forme cristalline A comprend des pics de diffraction caractéristiques aux angles 2θ suivants : 4,358°, 9,530°, 12,135°, 12,784°, 14,166°, 15,597°, 16,622°, 16,973°, 17,521°, 17,858°, 18,617°, 19,495°, 20,531°, 20,956°, 22,271°, 22,815°, 24,711°, 25,955°, 26,976°, 27,682°, 28,728°, 30,119°, 31,329°, 33,598°, et 35,500°.

6. Forme cristalline A du composé 1 selon la revendication 1, dans laquelle le diagramme de diffraction des rayons X sur poudre pour la forme cristalline A est tel que représenté à la FIG. 1 ;
ou, la courbe de calorimétrie différentielle à balayage pour la forme cristalline A est telle que représentée à la FIG. 2 ;
ou, la courbe d'analyse thermogravimétrique de la forme cristalline A est telle que représentée à la FIG. 3.

7. Forme cristalline A du composé 1 selon la revendication 1, dans laquelle la forme cristalline A présente une courbe de calorimétrie différentielle à balayage comprenant un pic endothermique ayant un début à 279,6 °C ± 3,0 °C ;
ou, la forme cristalline A présente une courbe d'analyse thermogravimétrique ayant une perte de poids de 0,59 % à 150,0 °C ± 3 °C.

8. Forme cristalline B du composé 1, 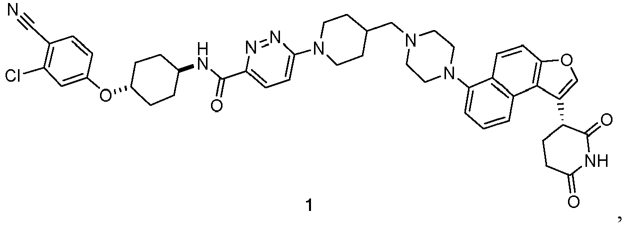 dans laquelle la forme cristalline B présente un diagramme de diffraction des rayons X sur poudre comprenant des pics de diffraction caractéristiques aux angles 2θ suivants : 10,655 ± 0,200°, 11,988 ± 0,200°, 16,055 ± 0,200°, 18,356 ± 0,200°, et 20,083 ± 0,200°.

9. Forme cristalline B du composé 1 selon la revendication 8, dans laquelle le diagramme de diffraction des rayons X sur poudre pour la forme cristalline B, représenté par des angles 2θ, comprend au moins 5, 6, 7 ou 8 pics de diffraction choisis parmi les suivants : 16,039 ± 0,200°, 16,923 ± 0,200°, 18,394 + 0,200°, 19,070 ± 0,200°, 19,445 ± 0,200°, 20,915 + 0,200°, 21,271 ± 0,200°, et 21,599 ± 0,200°.

10. Forme cristalline B du composé 1 selon la revendication 8, dans laquelle le diagramme de diffraction des rayons X sur poudre pour la forme cristalline B comprend des pics de diffraction caractéristiques aux angles 2θ suivants : 16,039 ± 0,200°, 16,923 ± 0,200°, 18,394 ± 0,200°, 19,070 + 0,200°, 19,445 ± 0,200°, 20,915 ± 0,200°, 21,271 + 0,200°, et 21,599 ± 0,200°;
de préférence, le diagramme de diffraction des rayons X sur poudre pour la forme cristalline B comporte des pics de diffraction caractéristiques aux angles 2θ suivants : 5,222 ± 0,200°, 10,412 ± 0,200°, 14,668 ± 0,200°, 16,039 ± 0,200°, 16,923 ± 0,200°, 18,394 ± 0,200°, 19,070 ± 0,200°, 19,445 ± 0,200°, 20,915 ± 0,200°, 21,271 ± 0,200°, 21,599 ± 0,200°, 23,951 ± 0,200°, et 25,463 ± 0,200°.

11. Forme cristalline B du composé 1 selon la revendication 8, dans laquelle le diagramme de diffraction des rayons X sur poudre pour la forme cristalline B, représenté par des angles 2θ, comprend au moins 10, 11, 12 ou 13 pics de diffraction choisis parmi les suivants : 5,222 ± 0,200°, 10,412 ± 0,200°, 14,668 ± 0,200°, 16,039 ± 0,200°, 16,923 ± 0,200°, 18,394 ± 0,200°, 19,070 ± 0,200°, 19,445 ± 0,200°, 20,915 ± 0,200°, 21,271 ± 0,200°, 21,599 ± 0,200°, 23,951 ± 0,200°, et 25,463 ± 0,200°.

12. Forme cristalline B du composé 1 selon la revendication 8, dans laquelle le diagramme de diffraction des rayons X sur poudre pour la forme cristalline B comprend des pics de diffraction caractéristiques aux angles 2θ suivants : 5,222°, 8,368°, 10,412°, 13,031°, 14,668°, 16,039°, 16,923°, 18,394°, 19,070°, 19,445°, 20,915°, 21,271°, 21,599°, 23,591°, 25,463°, 27,635°, et 31,559°.

13. Forme cristalline B du composé 1 selon la revendication 8, dans laquelle le diagramme de diffraction des rayons X sur poudre pour la forme cristalline B est tel que représenté sur la FIG. 4 ;
ou, la courbe de calorimétrie différentielle à balayage pour la forme cristalline B est telle que représentée à la FIG. 5 ;
ou, la courbe d'analyse thermogravimétrique de la forme cristalline B est telle que représentée à la FIG. 6.

14. Forme cristalline B du composé 1 selon la revendication 8, dans laquelle la forme cristalline B présente une courbe de calorimétrie différentielle à balayage comprenant un pic endothermique ayant une valeur maximale à 290,2 °C ± 3,0 °C ;
ou, la forme cristalline B présente une courbe d'analyse thermogravimétrique ayant une perte de poids de 1,75 % à 150,0 °C ± 3 °C.

15. Forme cristalline A du composé 1 selon les revendications 1 à 7 ou forme cristalline B du composé 1 selon les revendications 8 à 14, destinée à être utilisée dans le traitement d'une maladie associée à une chimère ciblant la protéolyse des récepteurs androgènes, dans laquelle la maladie associée à une chimère ciblant la protéolyse des récepteurs androgènes est de préférence un cancer de la prostate.
